# EUROPÄISCHE PATENTSCHRIFT

(11) **EP 2 678 321 B1**
(45) Veröffentlichungstag und Bekanntmachung des Hinweises auf die Patenterteilung: **05.10.2016**
(21) Anmeldenummer: 12701509.7
(22) Anmeldetag: 27.01.2012
(51) Int. Cl.: C07D 307/68, C08K 5/12

(54) **C11- C13 DIALKYLESTER DER FURANDICARBONSÄURE ALS WEICHMACHER**
C11 - C13 DIALKYLESTERS OF FURANDICARBONIC ACID AS PLASTICIZERS
ESTER C11-C13 DIALKYLE D'ACIDE FURANDICARBOXYLIQUE COMME PLASTIFIANTS

(30) Priorität: 24.02.2011 DE 102011004675
(43) Veröffentlichungstag der Anmeldung: 01.01.2014
(73) Patentinhaber: Evonik Degussa GmbH, 45128 Essen (DE)
(72) Erfinder: BECKER, Hinnerk Gordon, 45257 Essen (DE); GRASS, Michael, 45721 Haltern am See (DE)
(86) Internationale Anmeldenummer: PCT/EP2012/051304
(87) Internationale Veröffentlichungsnummer: WO 2012/113607

(56) Entgegenhaltungen:
- WO-A1-2011/023590
- WO-A1-2014/193634
- SANDERSON R D ET AL: "SYNTHESIS AND EVALUATION OF DIALKYL FURAN-2,5-DICARBOXYLATES AS PLASTICIZERS FOR PVC", JOURNAL OF APPLIED POLYMER SCIENCE, JOHN WILEY & SONS, INC, US, Bd. 53, Nr. 13, 26. September 1994 (1994-09-26), Seiten 1785-1793, XP000464476, ISSN: 0021-8995, DOI: 10.1002/APP.1994.070531308
- "Kunststoff Taschenbuch", ISBN: 9783446216051 * page 452 *

## Beschreibung

Die vorliegende Erfindung betrifft C₁₁ bis C₁₃ Dialkylester der Furandicarbonsäure und Gemische derselben.

Des Weiteren betrifft die Erfindung Weichmacher enthaltend C₁₁ bis C₁₃ Dialkylester der Furandicarbonsäure und Gemische derselben. Ein weiterer Gegenstand der Erfindung ist die Verwendung dieser Weichmacher in Polymeren insbesondere PVC, sowie ein Verfahren zur Herstellung des Weichmachers und die Verwendung der Weichmacher für verschiedene Anwendungen.

Polyvinylchlorid (PVC) ist eines der wirtschaftlich bedeutendsten Polymere und wird sowohl als Hart-PVC wie auch als Weich-PVC in vielfältigen Anwendungen eingesetzt. Wichtige Anwendungsbereiche sind beispielsweise Kabelummantelungen, Fußbodenbeläge, Tapeten und Rahmen für Kunststofffenster. Zur Erhöhung der Elastizität und zur besseren Verarbeitbarkeit werden dem PVC Weichmacher zugesetzt. Zu diesen üblichen Weichmachern gehören beispielsweise Phthalsäureester wie Di-2-Ethylhexylphthalat (DEHP), Disononylphthalat (DINP) und Diisodecylphthalat (DIDP). Wegen ihrer toxikologischen Eigenschaften ist man in vielen Fällen bemüht, Phthalsäureester durch andere Weichmacher zu ersetzen. Als alternative Weichmacher sind daher in neuerer Zeit beispielsweise Cylclohexandicarbonsäureester beschrieben worden wie Di-isononylcyclohexancarbonsäureester (DINCH).

Weiterhin wurden im Stand der Technik auch Ester der Terephthalsäure, insbesondere Di-2-ethylhexylterephthalat (DEHT oder DOTP) als alternative Weichmacher beschrieben.

In der WO 2009/095126 A1 werden Gemische von Di-isononylestern der Terephthalsäure, sowie Verfahren zu ihrer Herstellung beschrieben. Diese Weichmacher weisen einen durchschnittlichen Verzweigungsgrad der Isononylreste auf, der im Bereich von 1,0 bis 2,2 liegt und werden ebenfalls als Weichmacher für PVC eingesetzt.

Wegen der begrenzten Verfügbarkeit fossiler Rohstoffe, der damit verbundenen zukünftig absehbaren starken Preissteigerungen und der auch durch die Politik immer stärker geförderten Verwendung von nachwachsenden Rohstoffen sollten insbesondere solche Ester zukünftig gute Marktchancen haben, bei denen zumindest die Säurekomponente auf natürlich vorkommenden Ressourcen wie Zucker, Fetten oder Ölen basiert.

Viele Ester der Furandicarbonsäure, wie beispielsweise Di-n-butylfuran-2,5-dicarboxylat und Di-n-hexylfuran-2,5-dicarboxylat, sind bei Raumtemperatur kristalline Feststoffe, die sich aufgrund ihrer Feststoffeigenschaft nicht für die Herstellung von Plastisolen einsetzen lassen. So ist die Herstellung von Polymerpasten bzw. Plastisolen im technischen Maßstab nur mit flüssigen Weichmachern zu realisieren. Feste Weichmacher müssen vorher in entsprechenden Lösungsmitteln gelöst werden, was das Verfahren aufwendig und teuer macht.

Di(2-ethylhexyl)-, Di (2-octyl), Dihexyl- und Dibutyl-Derivate von Furandicarboxylaten werden in dem Artikel "Synthesis and Evaluation of Dialkyl Furan-2,5-Dicarboxylates as Plasticizers for PVC" von R.D. SAnderson, D.F. Schneider und I. Schreuder im Journal of Applied Polymer Science, Vol. 53, 1985 - 1793 (1994) beschrieben.

Furandicarboxylate mit höheren einwertigen Alkoholen als C8 sind noch nicht bekannt, und daher auch noch nicht als Weichmacher für Kunststoffe beschrieben. Für Anwendungen, bei denen hohe Anforderungen an das Emissionsverhalten gestellt werden, wie zum Beispiel Hochtemperaturkabel oder Automobil-Innenausstattung, gibt es daher kaum geeignete Weichmacher auf Basis nachwachsender Rohstoffe.

Bisher werden für diese Anwendungen auch die Ester der Phthalsäure verwendet, die jedoch aufgrund ihrer toxikologischen Eigenschaften als kritisch eingestuft werden und darüber hinaus nicht aus nachwachsenden Rohstoffen hergestellt werden können.

Ausgehend von diesem Stand der Technik liegt die technische Aufgabe der Erfindung darin, neue Substanzen zur Verfügung zu stellen, die auf einfache Weise aus nachwachsenden Rohstoffen hergestellt werden können und gute Weichmachereigenschaften im Vergleich zu den bisher verwendeten Weichmachern besitzen.

Die technische Aufgabe der Erfindung wird gelöst durch C₁₁ bis C₁₃ Dialkylester der Furandicarbonsäure.

Überraschenderweise wurde gefunden, dass C₁₁-C₁₃ Dialkylester der Furandicarbonsäure als Weichmacher oder als Bestandteil einer Weichmacherzusammensetzung für Kunststoffe, insbesondere für Polyvinylchorid (PVC), Polyvinylbutyral (PVB) und Polyalkylmethacrylat (PAMA), verwendet werden können und dort vorteilhafte Eigenschaften besitzen, verglichen zu den üblicherweise als Weichmachern verwendeten analogen Phthalsäureestern.

Die erfindungsgemäßen Weichmacher besitzen gegenüber den analogen Phthalatweichmachern des Standes der Technik niedrigere Dryblendzeiten. Dies bedeutet, dass eine geringere Mischzeit notwendig ist bei der Herstellung der Dryblends (= pulverförmige Mischungen aus Polymer, insbesondere PVC, und weiteren Formulierungsbestandteilen; die flüssigen Formulierungsbestandteile werden beim Mischvorgang von den Polymerpartikeln absorbiert) und sich damit ein Kostenvorteil gegenüber den Weichmachern des Standes der Technik ergibt. Ein weiterer Vorteil der erfindungsgemäßen Weichmacher liegt in der geringen Flüchtigkeit. Diese ist geringer als bei den entsprechenden Phthalatweichmachern, sodass das Ausschwitzen oder Ausdünsten des Weichmachers weniger stark auftritt und daher eine sichere Verarbeitung des Weichmachers im Polymer möglich ist. Weiterhin wurde festgestellt, dass die erfindungsgemäßen Weichmacher eine bessere weichmachende Wirkung besitzen und damit eine höhere Effizienz, wenn sie mit den entsprechenden Phthalatweichmachern des Standes der Technik verglichen werden.

Der Weichmacher enthält mindestens zwei isomere C₁₁-C₁₃ Dialkylfuran-2,5-dicarboxylate. Diese isomeren C₁₁-C₁₃ Dialkylfuran-2,5-dicarboxylate enthalten isomere C₁₁-C_{13'}Alkylgruppen. Diese sind in besonders bevorzugter Weise isomere C₁₁-C₁₃Alkylgruppen, ausgewählt aus der Gruppe unverzweigter Alkylgruppen, einfach verzweigter Alkylgruppen, zweifach verzweigter Alkylgruppen, dreifach verzweigter Alkylgruppen, vierfach verzweigter Alkylgruppen oder Mischungen davon.

In einer bevorzugten Ausführungsform ist keiner der isomeren C₁₁-C₁₃ Dialkylfuran-2,5-dicarboxylate in einem Anteil von mehr als 90 Gew.-% im Estergemisch enthalten. Es ist weiterhin bevorzugt, dass der Anteil an unverzweigten C₁₁-C₁₃ Alkylgruppen im Estergemisch in einem Bereich von 0,01 bis 80 Gew.-% liegt.

In einer ganz besonders bevorzugten Ausführungsform ist der Weichmacher dadurch gekennzeichnet, dass er Ditridecylfuran-2,5-dicarboxylat enthält. Diese Produkte eignen sich wegen ihrer besonders niedrigen Flüchtigkeit in ganz besonderer Weise zur Herstellung von Produkten mit hohen Anforderungen an lange Lebensdauer auf Grund geringer Verdampfungsneigung.

In einer weiteren bevorzugten Ausführungsform kann der Weichmacher zusätzliche andere Weichmacher enthalten, die insbesondere ausgewählt sind aus der Gruppe Alkylbenzoate, Dia-Ikyladipate, Glycerinester, epoxidierte Pflanzenöle; gesättigte oder ungesättigte Fettsäureester, welche auch partiell oder vollständig epoxidiert sein können; Citronensäuretrialkylester, acylierte Citronensäuretrialkylester, Trialkylmellitate, Glykoldibenzoate, Dialkylterephthalate, Dialkylphthalate, Isosorbidester, insbesondere Dialkanoylester des Isosorbit, Dialkylester der 1,2-, 1,3-oder 1,4-Cyclohexandicarbonsäure. Diese zusätzlichen Weichmacher sind im Einzelnen beispielsweise ausgewählt aus der folgenden Liste:
Phthalsäuredialkylester, bevorzugt mit 4 bis 13 C-Atomen in der Alkylkette; Trimellitsäuretrialkylester, bevorzugt mit 4 bis 10 C-Atomen in der Seitenkette; Adipinsäuredialkylester, bevorzugt mit 4 bis 13 C-Atomen; Terephthalsäuredialkylester, jeweils bevorzugt mit 4 bis 10 C-Atomen, insbesondere 7 bis 9 C-Atomen in der Seitenkette; 1,2-Cyclohexandisäurealkylester, 1,3-Cyclohexandisäurealkylester und 1,4-Cyclohexandisäure-alkylester, hierbei bevorzugt 1,2-Cyclohexandisäurealkyl-ester, jeweils bevorzugt mit 4 bis 13 Kohlenstoffatomen in der Seitenkette; Dibenzoesäurester von Glykolen; Alkylsulfonsäureester von Phenol mit vorzugsweise einem Alkylrest, der 8 bis 22 C-Atome enthält; Glycerinester, Citronensäuretriester mit freier oder carboxylierter OH-Gruppe und beispielsweise Alkylresten von 4 bis 10 C-Atomen, Alkylpyrrolidonderivate mit Alkylresten von 4 bis 18 C-Atomen sowie Benzoesäurealkylester, vorzugsweise mit 8 bis 13 C-Atomen in der Alkylkette. In allen Fällen können die Alkylreste linear oder verzweigt und gleich oder verschieden sein.

Besonders bevorzugt wird in den erfindungsgemäßen Mischungen kein *ortho*-Phthalat als zusätzlicher Weichmacher eingesetzt.

In einer besonderen Ausführungsform handelt es sich bei mindestens einem der in der erfindungsgemäßen Zusammensetzung verwendeten zusätzlichen Weichmacher um ein Trimellitsäuretrialkylester. Bevorzugt weist dieser Trimellitsäuretrialkylester Esterseitenketten mit 4 bis 10 Kohlenstoffatomen auf, wobei die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit minimal 8 Kohlenstoffatomen pro Estergruppe, insbesondere bevorzugt um eine Gruppe mit minimal 9 Kohlenstoffatomen und ganz besonders bevorzugt um eine Gruppe mit minimal 10 Kohlenstoffatomen.

In einer weiteren besonderen Ausführungsform handelt es sich bei mindestens einem der in der erfindungsgemäßen Zusammensetzung verwendeten zusätzlichen Weichmacher um einen Adipinsäuredialkylester. Bevorzugt weist dieser Adipinsäuredialkylester Esterseitenketten mit 4 bis 13 Kohlenstoffatomen auf, wobei auch hier die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit minimal 8 Kohlenstoffatomen pro Estergruppe, insbesondere bevorzugt um eine Gruppe mit minimal 10 Kohlenstoffatomen und ganz besonders bevorzugt um eine Gruppe mit 13 Kohlenstoffatomen. Insbesondere handelt es sich bei mindestens einem der verwendeten Adipinsäuredialkylester um Di-ethylhexyladipat, Di-isononyladipat, Diisodecyladipat, Di-propylheptyladipat oder Di-isotridecyladipat.

In einer weiteren besonderen Ausführungsform handelt es sich bei mindestens einem der in der erfindungsgemäßen Zusammensetzung verwendeten zusätzlichen Weichmacher um einen Terephthalsäuredialkylester. Bevorzugt weist dieser Terephthalsäuredialkylester Esterseitenketten mit 4 bis 10 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit minimal 4 Kohlenstoffatomen pro Estergruppe, insbesondere bevorzugt um eine Gruppe mit minimal 9 Kohlenstoffatomen und ganz besonders bevorzugt um eine Gruppe mit 10 Kohlenstoffatomen. Insbesondere handelt es sich bei mindestens einem der verwendeten Terephthalsäuredialkyl-ester um Di-*n*-heptylterephthalat, Di-*iso*-heptylterephthalat, Di-*n-*butylterephthalat, Di-(3-methylbutyl)terephthalat, Di-*n*-pentylterephthalat, Di-2-ethylhexylterephthalat oder Di-isononylterephthalat.

In einer weiteren besonderen Ausführungsform handelt es sich bei mindestens einem der in der erfindungsgemäßen Zusammensetzung verwendeten zusätzlichen Weichmacher um einen Dia-Ikylester der Cyclohexandicarbonsäure, besonders bevorzugt um einen Dialkylester der 1,2-Cyclohexandicarbonsäure. Bevorzugt weist dieser Cyclohexandicarbonsäuredialkylester Esterseitenketten mit 4 bis 13 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit minimal 5 Kohlenstoffatomen pro Estergruppe, insbesondere bevorzugt um eine Gruppe mit minimal 9 Kohlenstoffatomen und ganz besonders bevorzugt um eine Gruppe mit minimal 10 Kohlenstoffatomen. Insbesondere handelt es sich bei mindestens einem der verwendeten Cyclohexandicarbonsäuredialkylester um 1,2-Cyclohexansäuredi-*n*-pentylester, 1,2-Cyclohexan-säuredi-*n*-heptylester, 1,2-Cyclohexansäuredi-*iso*-heptylester, oder 1,2-Cyclohexansäuredi-(3-methylbutyl)ester, 1,2-Cyclohexandi-2-ethylhexylester, 1,3-Cyclohexandi-2-ethylhexylester, 1,4-Cyclohexandi-2-ethylhexylester,sowie 1,2-Cyclohexandicarbonsäurediisononylester, 1,3- Cyclohexandicarbonsäurediisononylester und 1,4-Cyclohexandicarbonsäurediisononylester.

In einer weiteren besonderen Ausführungsform handelt es sich bei mindestens einem der in der erfindungsgemäßen Zusammensetzung verwendeten zusätzlichen Weichmacher um einen Glycerinester, besonders bevorzugt um einen Glycerintriester. Die Estergruppen können dabei sowohl von aliphatischer als auch aromatischer Struktur sein. Bevorzugt weist dieser Glycerinester Esterseitenketten mit 1 bis 9 Kohlenstoffatomen auf, wobei wiederum die Estergruppen entweder die gleiche oder eine voneinander verschiedene Anzahl an Kohlenstoffatomen aufweisen können, sowie linear oder verzweigt, gesättigt oder ungesättigt sein können oder auch ein oder mehrere Epoxideinheiten enthalten können. Besonders bevorzugt handelt es sich bei mindestens einer der vorhandenen Estergruppen um eine Gruppe mit minimal 2 Kohlenstoffatomen pro Estergruppe, insbesondere bevorzugt um eine Gruppe mit minimal 8 Kohlenstoffatomen und ganz besonders bevorzugt um eine Gruppe mit 9 Kohlenstoffatomen. Weiterhin bevorzugt können Glycerinester mit Esterseitenketten mit 1 bis 24 Kohlenstoffatomen eingesetzt werden, wobei wiederum die Estergruppen entweder die gleiche oder eine voneinander verschiedene Anzahl an Kohlenstoffatomen aufweisen können. Besonders bevorzugt handelt es sich bei einer der Estergruppen um Hydroxystearinsäure, wobei die Hydroxyfunktion bevorzugt ebenfalls verestert ist, besonders bevorzugt durch eine Acetylgruppe. Weiterhin bevorzugt handelt es sich bei mindestens einer der Estergruppen um Laurinsäure.

In einer weiteren besonderen Ausführungsform handelt es sich bei mindestens einem der in der erfindungsgemäßen Zusammensetzung verwendeten zusätzlichen Weichmacher um einen Citronensäuretriester mit freier oder carboxylierter OH-Gruppe. Die Estergruppen können dabei auch hier sowohl von aliphatischer als auch aromatischer Struktur sein. Insbesondere bevorzugt handelt es sich um einen Citronensäuretrialkylester mit carboxylierter OH-Gruppe. Bevorzugt weist dieser Citronensäuretrialkylester Esterseitenketten mit 2 bis 10 Kohlenstoffatomen auf, wobei wiederum die Estergruppen entweder die gleiche oder eine voneinander verschiedene Anzahl an Kohlenstoffatomen aufweisen können. Besonders bevorzugt handelt es sich bei mindestens einer der vorhandenen Estergruppen um eine Gruppe mit minimal 4 Kohlenstoffatomen pro Estergruppe, insbesondere bevorzugt um eine Gruppe mit minimal 8 Kohlenstoffatomen und ganz besonders bevorzugt um eine Gruppe mit minimal 9 Kohlenstoffatomen. Insbesondere handelt es sich bei mindestens einem der verwendeten Citronensäureestern um Acetyl-tributylcitrat, Acetyl-tri-*n*-butylcitrat, Acetyl-tri-*n*-pentylcitrat, Acetyl-tri-*iso*-heptylcitrat, Acetyl-tri-2-ethylhexylcitrat oder Acetyl-triisononylcitrat.

In einer bevorzugten Ausführungsform liegt das Massenverhältnis aus eingesetzten zusätzlichen Weichmachern und den C₁₁ bis C₁₃ Dialkylester der Furandicarbonsäure zwischen 1:20 bis 20:1, besonders bevorzugt von 1:20 bis 10:1 und ganz besonders bevorzugt von 1:20 bis 5:1.

Neben dem Weichmacher selbst wird auch ein Verfahren zu dessen Herstellung beansprucht. Ein solches Verfahren umfasst die Verfahrensschritte:
a) Umsetzung eines stöchiometrischen Überschusses von C₁₁-C₁₃-Alkoholen, optional in Gegenwart eines Katalysators, mit Furandicarbonsäure oder einem entsprechend geeigneten Derivat der Furandicarbonsäure
b) Abtrennen des überschüssigen Alkohols nach vollständiger Umsetzung der Furandicarbonsäure oder der des entsprechend geeigneten Derivats zum C₁₁-C₁₃-Dialkylester der Furandicarbonsäure und
c) Aufarbeitung des Reaktionsgemisches zur Gewinnung des erfindungsgemäßen Produktes in hoher Reinheit.

Ein alternatives Verfahren zur Herstellung eines C₁₁ bis C₁₃ Dialkylesters der Furandicarbonsäure umfasst die folgenden Verfahrensschritte:
a) In Kontakt bringen von 5-Hydroxymethylfurfural und / oder mindestens eines Furanderivates mit einem oder mehreren aliphatischen Alkoholen mit 11-13 Kohlenstoffatomen, sowie mindestens einem Katalysator und mindestens einer sauerstoffhaltigen Komponente,
b) Temperieren der beschriebenen Reaktionsmischung auf eine Temperatur > 0 °C und Durchführung einer oxidativen Veresterung, wobei unter dem Begriff der "oxidativen Veresterung" die (beliebige) Kombination aus Oxidation und Veresterung und gegebenenfalls der Abspaltung einer Schutzgruppe von dem Furanderivat in bevorzugt einem Verfahrensschritt, insbesondere bevorzugt in einem Reaktionsraum, zu verstehen ist.

Die erfindungsgemäßen C₁₁ bis C₁₃ Dialkylester der Furandicarbonsäure können hergestellt werden durch Veresterung der Furandicarbonsäure oder durch Umesterung beispielsweise aus den Methylestern der Furandicarbonsäure.

Das erfindungsgemäße Verfahren zur Herstellung isomerer C₁₁-C₁₃-Dialkylester der 2,5-Furandicarbonsäure zeichnet sich dadurch aus, dass 2,5-Furandicarbonsäure oder ein kürzerkettiger Dialkylester dieser Verbindung, bevorzugt der Dimethylester, mit einem Gemisch isomerer C₁₁-C₁₃-Alkohole unter optionaler Verwendung eines Katalysators umgesetzt wird. Weiterhin kann auch das 2,5-Furandicarbonsäure-dichlorid, welches durch Umsetzung der FDCA mit Chlorierungsmitteln wie beispielweise Thionylchlorid erhalten werden kann, als Ausgangsstoff zur Herstellung der Furandicarbonsäureester eingesetzt werden.

Vorzugsweise wird ein Gemisch isomerer C₁₁-C₁₃-Alkohole eingesetzt, insbesondere ein Gemisch isomerer C₁₂-C₁₃-Alkohole und ganz besonders bevorzugt ein Gemisch isomerer C₁₃-Alkohole eingesetzt.

### Herstellung der isomeren Alkoholgemische

Verfahren zur Herstellung der entsprechenden Alkoholgemische sind literaturbekannt. Das wichtigste Verfahren zur Herstellung stellt die Hydroformylierung entsprechender Olefine oder Olefingemische mit der im Vergleich zum Alkohol um ein C-Atom niedrigeren C-Zahl mit nachfolgender Hydrierung zu den entsprechenden Alkoholgemischen dar. Im Falle der Herstellung von Tridecylalkohol erfolgt die Herstellung beispielsweise aus Tributen oder Tetrapropylen (C₁₂-Olefin). Hydroformylierungsverfahren werden beschrieben in der DE199 55 593 A1 und der EP 1515934 A1.

Die zur Hydroformylierung eingesetzten Olefine können alle die gleiche C-Atom-Anzahl haben, wie zum Beispiel die aus dem Octol-Prozess als Nebenprodukt erhältlichen C₁₂-Olefine, die auch unter der Bezeichnung Tributen bekannt sind. Ein am Markt bekanntes Gemisch isomerer C₁₃-Alkohole ist das von Sasol vertriebene Marlipal 013 oder das Isotridecanol N von BASF.

Weiterhin können diese Olefine auch aus Destillationsschnitten stammen, die Olefine mit verschiedenen C-Atomen enthalten wie zum Beispiel aus dem Polygasprozess bekannt und beispielsweise von der Firma ExxonMobil Chemical hergestellt und in die Alkohole überführt werden (z.B. Exxal 13). Der hieraus erhältliche Phthalatweichmacher ist als JAYFLEX DTDP seit vielen Jahren im Markt bekannt.

Neben den durch Hydroformylierung herstellbaren Alkoholen sind auch weitere Verfahren bekannt. Beispielsweise seien hier der Alfol-Prozess zur Herstellung geradzahliger, linearer Alkohole aber auch die Hydrierung von Fettsäuren oder Fettsäureestern, insbesondere Fettsäuremethylester genannt. Eine Übersicht über Verfahren zur Hydrierung der Aldehyde zu den entsprechenden Alkoholen findet sich beispielweise in EP 1749572 A1.

### Veresterung

Zur Herstellung der erfindungsgemäßen Ester wird entweder 2,5-Furandicarbonsäure oder ein reaktives Derivat wie beispielsweise das entsprechende Dichlorid oder der Dimethylester mit einem Gemisch isomerer C₁₁-C₁₃-Alkohole umgesetzt. Bevorzugt erfolgt die Veresterung ausgehend von Furandicarbonsäure oder Furandicarbonsäuredimethylester und besonders bevorzugt von Furandicarbonsäuredimethylester und den entsprechenden C₁₁-C₁₃-Alkoholen mit Hilfe eines Katalysators.

Die Veresterung der Furandicarbonsäure mit einem C₁₁-C₁₃-Alkoholgemisch zu den entsprechenden Estern kann autokatalytisch oder katalytisch, beispielsweise mit Brönstedt- oder Lewissäuren durchgeführt werden. Ganz gleich welche Art der Katalyse gewählt wird, es entsteht immer ein temperaturabhängiges Gleichgewicht zwischen den Einsatzstoffen (Säure und Alkohol) und den Produkten (Ester und Wasser). Um das Gleichgewicht zu Gunsten des Esters zu verschieben, kann ein Schleppmittel eingesetzt werden, mit dessen Hilfe das Reaktionswasser aus dem Ansatz entfernt wird. Da die zur Veresterung eingesetzten Alkoholgemische niedriger als die Furandicarbonsäure, deren reaktive Derivate und deren Ester sieden und mit Wasser eine Mischungslücke aufweisen, werden sie häufig als Schleppmittel eingesetzt, das nach Wasserabtrennung wieder in den Prozess zurückgeführt werden kann.

Der zur Bildung des Esters eingesetzte Alkohol bzw. das isomere C₁₁-C₁₃-Gemisch, das gleichzeitig als Schleppmittel dient, wird im Überschuss, bevorzugt im Überschuss von 5 bis 120 Massen-%, insbesondere von 10 bis 80 Massen-%, der zur Bildung des Esters notwendigen Menge eingesetzt.

Als Veresterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität oft erst bei Temperaturen oberhalb 180 °C erreichen. Hierbei ist jedoch zu beachten, dass die Furandicarbonsäure bei Temperaturen oberhalb von 190 °C zur Abspaltung von CO₂ neigt und sich hieraus dann die Monocarbonsäure bildet, welche natürlich dann nicht mehr zum Zielprodukt umgesetzt werden kann. Bei der Verwendung von Furandicarbonsäuredimethylester bestehen diese Nachteile nicht.

Die Metallkatalysatoren werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirconiumester wie Tetrabutylzirconat.

Die Katalysatorkonzentration hängt von der Art des Katalysators ab. Bei den bevorzugt eingesetzten Titanverbindungen beträgt diese 0,005 bis 2,0 Massen-% bezogen auf das Reaktionsgemisch, insbesondere 0,01 bis 0,5 Massen-%, ganz besonders 0,01 bis 0,1 Massen-%.

Die Reaktionstemperaturen liegen bei Verwendung von Titankatalysatoren zwischen 160 °C und 270 °C, vorzugsweise bei 160 °C bis 200 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers günstig, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei vermindertem Druck durchgeführt. Beispielsweise wird bei der Umsetzung von FDCA mit einem Gemisch isomerer Tridecanole in einem Temperaturbereich von 160 °C bis 190 °C im Druckbereich von 0,1 MPa bis 0,001 MPa gearbeitet.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereitstehenden Alkohol zu ersetzen.

Die Rohestergemische, die neben dem/den Ester(n), Alkohol, Katalysator oder dessen Folgeprodukten und gegebenenfalls Nebenprodukte enthalten, werden nach an sich bekannten Verfahren aufgearbeitet. Die Aufarbeitung umfasst dabei folgende Schritte: Abtrennung des überschüssigen Alkohols und gegebenenfalls Leichtsieder, Neutralisation der vorhandenen Säuren, optional eine Wasserdampfdestillation oder eine Strippung mit Inertgas, Umwandlung des Katalysators in einen leichtfiltrierbaren Rückstand, Abtrennung der Feststoffe und gegebenenfalls eine Trocknung. Dabei können je nach angewendetem Aufarbeitungsverfahren die Reihenfolge dieser Schritte verschieden sein.

Optional kann das Gemisch der C₁₁ bis C₁₃ Dialkylester der Furandicarbonsäure aus dem Reaktionsgemisch, gegebenenfalls nach Neutralisation des Ansatzes, destillativ abgetrennt werden.

### Umesterung

Alternativ können die erfindungsgemäßen C₁₁ bis C₁₃ Dialkylester der Furandicarbonsäure durch Umesterung eines Furan-2,5-dicarbonsäurediesters mit einem C₁₁ bis C₁₃-Alkoholgemisch gewonnen werden. Als Edukte werden Furan-2,5-dicarbonsäurediester eingesetzt, deren am O-Atom der Estergruppe gebundenen Alkylreste 1-10 C-Atome aufweisen. Diese Reste können aliphatisch, geradkettig oder verzweigt, alicyclisch oder aromatisch sein. Eine oder mehrere Methylengruppen dieser Alkyl-Reste können durch Sauerstoff substituiert sein. Es ist zweckmäßig, dass die dem Eduktester zugrunde liegenden Alkohole niedriger sieden als das eingesetzte C₁₁-C₁₃-Alkoholgemisch. Ein bevorzugter Einsatzstoff ist Furan-2,5-dicarbonsäuredimethylester.

Die Umesterung wird katalytisch, beispielsweise mit Brönstedt- oder Lewissäuren oder Basen, durchgeführt. Ganz gleich welcher Katalysator eingesetzt wird, es entsteht immer ein temperaturabhängiges Gleichgewicht zwischen den Einsatzstoffen Dialkylester der Furandicarbonsäure und C₁₁-C₁₃-Alkoholgemisch und den Produkten C₁₁ bis C₁₃ Dialkylester der Furandicarbonsäure und freigesetzter Alkohol. Um das Gleichgewicht zu Gunsten der C₁₁ bis C₁₃ Dialkylester der Furandicarbonsäure zu verschieben, wird der aus dem Eduktester entstehende Alkohol aus dem Reaktionsgemisch abdestilliert. Es ist auch hier zweckmäßig, das C₁₁- bis C₁₃-Alkoholgemisch im Überschuss einzusetzen.

Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirconium, die als feinverteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat, Tetraisotridecylorthotitanat sowie Zirconiumester wie Tetrabutylzirconat oder Tetraisotridecylzirconat.

Weiterhin können basische Katalysatoren, wie beispielsweise Oxide, Hydroxide, Hydrogencarbonate, Carbonate oder Alkoholate von Alkali- oder Erdalkalimetallen verwendet werden. Aus dieser Gruppe werden bevorzugt Alkoholate, wie beispielsweise Natriummethylat eingesetzt. Alkoholate können auch in situ aus einem Alkalimetall und einem C₁₁- bis C₁₃-Alkohol hergestellt werden.

Die Katalysatorkonzentration hängt von der Art des Katalysators ab. Sie liegt üblicherweise zwischen 0,005 bis 2,0 Massen-% bezogen auf das Reaktionsgemisch.

Die Reaktionstemperaturen für die Umesterung liegen üblicherweise zwischen 100 °C und 250 °C. Sie müssen mindestens so hoch sein, dass der aus dem Eduktester entstehende Alkohol bei dem vorgegebenen Druck, meistens Normaldruck, aus dem Reaktionsgemisch abdestillieren kann.

Die Umesterungsgemische können genauso wie für die Veresterungsgemische beschrieben aufgearbeitet werden, optional kann auf eine Neutralisation verzichtet werden.

Es wird die Verwendung der zuvor beschriebenen erfindungsgemäßen Ester in Klebstoffen, Dichtungsmassen, Beschichtungsmassen, Lacken, Farben, Plastisolen, Kunstleder, Fußbodenbeläge, Unterbodenschutz, Gewebebeschichtungen, Kabeln oder Drahtisolierungen, Extrusionsartikeln, sowie in Folien, insbesondere für den Automobilinnenbereich und auch in Tapeten oder Tinten beansprucht.

Die erfindungsgemäßen Weichmacher können zur Modifizierung von Polymeren eingesetzt werden. Diese Polymere sind vorzugsweise ausgewählt aus der Gruppe bestehend aus:
Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat-Copolymere (ASA), Styrolacrylonitril-Copolymere (SAN), Acrylonitril-Butadien-Styrol-Copolymere (ABS), Styrol-Maleinsäureanhydrid-Copolymere (SMA), Styrol-Methacrylsäure-Copolymere, Polyolefine und/oder Polyolefincopolymere, insbesondere Polyethylen (PE) oder Polypropylen (PP), thermoplastische Polyolefine (TPO), Polyethylen-Vinylacetat-Copolymere (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere o-der deren monomeren Einheiten aufweisen. Vorzugsweise weisen die erfindungsgemäßen Polymere PVC oder Homo- oder Copolymere auf Basis von Ethylen, Propylen, Butadien, Vinylacetat, Glycidylacrylat, Glycidylmethacrylat, Methacrylaten, Ethylacrylaten, Butylacrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf.

Besonders bevorzugt ist das Polymer Polyvinylacetat, Polyalkylmethacrylat oder ein Copolymer von Vinylchlorid mit einem oder mehreren Monomeren ausgewählt aus der Gruppe bestehend aus Vinylidenchlorid, Vinylacetat, Vinylpropionat, Vinylbutyrat, Vinylbenzoat, Methylacrylat, Ethylacrylat, oder Butylacrylat.

Bevorzugt enthält das Polymer als PVC-Typ Suspensions-, Masse-, Mikrosuspensions- oder Emulsions-PVC.

Bezogen auf 100 Massenteile Polymer enthalten die erfindungsgemäßen Polymere vorzugsweise von 5 bis 200, bevorzugt von 10 bis 150 Massenteile an Weichmacher.

In bestimmten Fällen kann das Verhältnis von Weichmacher zu Polymer im Bereich von 1:15 bis 15:1 liegen.

Die Polymere können neben den genannten Bestandteilen Additive enthalten, die insbesondere ausgewählt sind aus der Gruppe bestehend aus Füllstoffen, Pigmenten, Thermostabilisatoren, Costabilisatoren, UV-Stabilisatoen, Antioxidantien, Viskositätsregulierer, Flammschutzmittel und Gleitmittel.

Die Thermostabilisatoren neutralisieren u.a. während und/oder nach der Verarbeitung des PVCs abgespaltene Salzsäure und verhindern einen thermischen Abbau des Polymeren. Als Thermostabilisatoren kommen alle üblichen PVC-Stabilisatoren in fester und flüssiger Form in Betracht, beispielsweise auf Basis von Ca/Zn, Ba/Zn, Pb, Sn oder organischer Verbindungen (OBS), sowie auch säurebindende Schichtsilikate wie Hydrotalcit. Die erfindungsgemäßen Gemische können einen Gehalt von 0,5 bis 12 bevorzugt 1 bis 10, besonders bevorzugt von 1,5 bis 8 Massenteilen pro 100 Massenteilen Polymer an Thermostabilisator aufweisen.

Als sogenannte Costabilisatoren (d.h. Substanzen, die die Wirkung der Thermostabilisatoren verlängern, verbessern und/oder ergänzen), können z.B. Pflanzenölderivate wie z.B. epoxidiertes Sojabohnenöl oder epoxidiertes Leinöl eingesetzt werden.

Als Pigmente können im Rahmen der vorliegenden Erfindung sowohl anorganische als auch organische Pigmente eingesetzt werden. Der Gehalt an Pigmenten liegt zwischen 0,01 bis 10 Massen-%, bevorzugt 0,05 bis 5 Massen-%, besonders bevorzugt 0,1 bis 3 Massen-% pro 100 Massenteilen Polymer. Beispiele für anorganische Pigmente sind TiO₂, CdS, CoO/Al₂O₃, Cr₂O₃. Bekannte organische Pigmente sind beispielsweise Azofarbstoffe, Phthalocyaninpigmente, Dioxazinpigmente sowie Anilinpigmente.

Die Polymere können alle dem Stand der Technik entsprechenden Füllstoffe enthalten. Beispiele solcher Füllstoffe sind mineralische und/oder synthetische und/oder natürliche, organische und/oder anorganische Materialien, wie z. B. Calciumoxid, Magnesiumoxid, Calciumcarbonat, Bariumsulfat, Siliziumdioxid, Schichtsilikat, Industrieruß, Bitumen, Holz (z. B. pulverisiert, als Granulat, Mikrogranulat, Fasern usw.), Papier, Natur- und/oder Synthetikfasern usw. Besonders bevorzugt handelt es sich bei mindestens einem der eingesetzten Füllstoffe um ein Calciumcarbonat oder ein Calcium-Magnesium-Carbonat.

Die erfindungsgemäßen Ester liegen bevorzugt als Flüssigkeit, insbesondere als pumpbare Flüssigkeit vor.

Die erfindungsgemäßen Weichmacher können in Klebstoffen, Dichtungsmassen, Lacken, Farben, Plastisolen, Kunstleder, Fußbodenbeläge, Unterbodenschutz, Gewebebeschichtungen, Kabeln, Tapeten oder Tinten verwendet werden. Besonders bevorzugt ist die Anwendung in Hochtemperaturkabeln und Kfz-Innenausstattung wie Folien für Armaturenbretter.

Die folgenden Beispiele sollen die Erfindung erläutern, ohne ihre Anwendungsbreite einzuschränken, die sich aus der Beschreibung und den Ansprüchen ergibt.

### BEISPIELE

### Beispiel 1: Synthese von Furan-2,5-dicarbonsäure-diisotridecylester

In einem 4-Liter Rührkolben mit Wasserauskreiser mit aufgesetztem Intensivkühler, Rührer, Tauchrohr, Tropftrichter und Thermometer wurden 234 g (1,5 Mol) Furan-2,5-dicarbonsäure, 0,59 g (0,25 Massen-% bezogen auf Furan-2,5-dicarbonsäure) Tetrabutyl-orthotitanat und 1200 g (6 Mol) eines über den OCTOL-Prozess hergestellten iso-Tridecanols (Marlipal 013, Fa. Sasol) vorgelegt und bei 170 °C verestert. Nach 26 Stunden war die Reaktion beendet und danach wurde bis 210 °C und 3 mbar der überschüssige Alkohol abdestilliert. Anschließend wurde auf 80 °C abgekühlt und mit 2 ml einer 10 Massen-%igen wässrigen NaOH-Lösung neutralisiert. Im Anschluss wurde bei 200°C und einem Druck von 30 mbar eine Reinigung durch Strippen mit Stickstoff durchgeführt. Dazu wurde bei maximalem Vakuum (1 mbar bei einer Saugleistung von 5 m3/h) der Druck über den Stickstoffstrom eingestellt. Danach wurde der Ansatz nach Abkühlen auf 100 °C filtriert. Laut GC ergab sich ein Estergehalt von >99,5 %.

### Beispiel 2 (Vergleichsbeispiel): Synthese von Phthalsäure-diisotridecylester

In einem 4-Liter Rührkolben mit Wasserauskreiser mit aufgesetztem Intensivkühler, Rührer, Tauchrohr, Tropftrichter und Thermometer wurden 592 g (4 Mol) Phthalsäureanhydrid (Fluka), 0,71 g (0,25 Massen-% bezogen auf Phthalsäureanhydrid) Tetrabutyl-orthotitanat und 2000 g (10 Mol) eines über den OCTOL-Prozess hergestellten iso-Tridecanols (Marlipal 013, Fa. Sasol) vorgelegt und bis 240 °C verestert. Nach 3 Stunden war die Reaktion beendet und danach wurde bis 210 °C und 3 mbar der überschüssige Alkohol abdestilliert. Anschließend wurde auf 80 °C abgekühlt und mit 6 ml einer 10 Massen-%igen wässrigen NaOH-Lösung neutralisiert. Im Anschluss wurde bei 200 °C eine Wasserdampfdestillation (8 Massen-% Wasser bezogen auf die Menge an Ester) durchgeführt. Danach wurde der Ansatz nach Abkühlen auf 100 °C filtriert. Laut GC ergab sich ein Estergehalt von >99,5 %.

### Beispiel 3 (Vergleichsbeispiel): Synthese von Terephthalsäure-diisotridecylester

Beispiel 3 wurde wie Beispiel 2 durchgeführt jedoch mit dem Unterschied, dass 664 g (4 Mol) Terephthalsäure (Merck) anstatt Phthalsäureanhydrid eingesetzt wurden. Entsprechend der geänderten Säuremenge wurde auch die Katalysatormenge angepasst. Laut GC ergab sich ein Estergehalt von >99,5 %.

### Beispiel 4: Bestimmung der Lösetemperatur

Die Lösetemperatur ist ein wichtiger Hinweis auf die Gelierfähigkeit eines Weichmachers.

Beschreibung der Prüfung:
In einem 150 ml Becherglas werden 96 g des entsprechenden Weichmachers der Beispiele 1 bis 3 und 4 g des PVC's Lacovyl PB 1704 H (Fa. Arkema) eingewogen. Zu der Mischung werden ein Magnetrührfisch und ein an einem Stativ befestigtes Innenthermometer (Bereich: 0 °C - 250 °C, Anzeigegenauigkeit: 0,5 °C) gegeben. Mit einem Draht oder Klebeband wird an der Rückseite des Glases ein Papierstreifen, der den Schriftzug "Lösetemperatur" in der Schriftart "Times New Roman", Schriftgröße 12, so befestigt, dass der Schriftzug durch das Becherglas hindurch zu sehen ist.

Danach wird die Heizplatte eines beheizbaren Laborrührgeräts (MR-Hei-Standard) auf 200 °C und die Drehzahl auf 600 U/min eingestellt. Nach Erreichen einer Innentemperatur der Flüssigkeit von 140 °C wurde die Solltemperatur nochmals auf 250 °C hochgeregelt. Die Lösetemperatur ist dann erreicht, wenn der Schriftzug durch die Flüssigkeit gerade klar lesbar geworden ist

### Ergebnis: (gerundeter Mittelwert von zwei Messungen)

| | |
|---|---|
| Beispiel 1 (Furanoat): | 159 °C |
| Beispiel 2 (Phthalat): | 161 °C |
| Beispiel 3 (Terephthalat): | größer 185 °C (Abbruch) |

Die Lösetemperatur des Terephthalats konnte nicht bestimmt werden, da oberhalb von 185°C mit einer PVC-Zersetzung zu rechnen ist und damit der Versuch abgebrochen wurde.

Das erfindungsgemäße Furanoat zeigt die niedrigste Lösetemperatur. Dies bedeutet, dass das erfindungsgemäße Furanoat eine niedrigere Verarbeitungstemperatur besitzt als die Weichmacher der Beispiele 2 und 3.

### Beispiel 5: Herstellung der Dryblends, Messung der Weichmacheraufnahme und des Drehmoments

Die mit den erfindungsgemäßen Estern erzielbaren vorteilhaften Eigenschaften sollen im Folgenden exemplarisch an Trockenmischungen, die als Dryblends bezeichnet werden, und den aus diesen erhältlichen Halbzeugen aufgezeigt werden.

Die hergestellten Rezepturen mit den Weichmachern der Beispiele 1 bis 3 sind in der nachfolgenden Tabelle 1 dargestellt. Hiermit lassen sich insbesondere Kabel- und Drahtisolierungen herstellen.

**Tabelle 1: Rezepturen der Trockenmischungen**

| (Alle Angaben in Massenteilen) | | | |
|---|---|---|---|
| **Trockenmischung** | **1*** | **2**** | **3**** |
| Solvin S 271 PC | 100 | 100 | 100 |
| Diisotridecylfurandicarboxylat | 50 | | |
| Diisotridecylphthalat | | 50 | |
| Diisotridecylterephthalat | | | 50 |
| OMYA BSH | 20 | 20 | 20 |
| Baeropan MC 8890 KA/2 | 8 | 8 | 8 |

| | | | |
|---|---|---|---|
| ** = Vergleichsbeispiel * = Erfindungsgemäß | | | |

Die verwendeten Stoffe und Substanzen werden im Folgenden näher erläutert:
**Solvin S 271 PC:** Suspensions-PVC mit einem K-Wert (Bestimmt nach DIN EN ISO 1628-2) von 71; Fa. SOLVIN S.A.
**OMYA BSH: mineralischer** Füllstoff auf Basis Calciumcarbonat, Fa. OMYA
**Baeropan MC 8890 KA/2:** Thermostabilisator auf Basis Ca/Zn für Hochtemperatur-Anwendungen, Fa. Baerlocher

Die Herstellung der Dryblends erfolgte in einem Brabender Planetenmischer. Ein mit vollentsalztem Wasser gefüllter Thermostat (Fa. Lauda RC6) sorgte für die Temperierung des Mischbehälters am Planetenmischer. Ein PC zeichnete die vom Mischer gesendeten Daten über ein Datenkabel in der Software "Winmix" auf.

Über die Software "Winmix" wurden folgende Parameter am Brabender Planetenmischer eingestellt.

| | |
|---|---|
| Drehzahlprogramm: | Aktiv |
| Profil: | Drehzahl 50 U/min; Haltezeit: 9 min; Anstiegszeit: 1 min Drehzahl 100 U/min; Haltezeit: 20 min |
| Knetertemperatur: | 88 °C |
| Messbereich: | 2 Nm |
| Dämpfung: | 3 |

Am Thermostat wurde eine Temperatur von 90 °C eingestellt und über eine Schlauchverbindung wurde der Mischbehälter am Brabender temperiert. Die Temperatur im Mischbehälter betrug nach einstündiger Temperierzeit 88 °C. Nachdem der Planetenmischer eine Eigenkalibrierung durchgeführt hatte, wurden die festen Bestandteile (PVC, Füllstoff, Stabilisator), welche vorher auf einer Waage (Fa. Mettler Typ XS6002S) in vierfacher Menge in einen PE-Becher einwogen wurden, dem Mischgefäß, über einen Feststofftrichter und den vorhandenen Einfüllstutzen am Brabender Mischgefäß, zugeführt. Das Programm wurde gestartet und die Pulvermischung wurde 10 Minuten im Mischgefäß gerührt und temperiert, ehe die flüssigen Bestandteile, welche ebenfalls in vierfacher Menge auf der Waage in einem PE-Becher ausgewogen wurden, über einen Flüssigkeitstrichter und dem vorhandenen Einfüllstutzen am Brabender Mischgefäß, zugeführt wurden. Die Mischung wurde weitere 20 Minuten im Planetenmischer gerührt. Nach Beendigung des Programms wurde die fertige Trockenmischung (Pulver) entnommen. Das übertragende Drehmoment-Zeit Diagramm wurde über die BRABENDER-Software ausgewertet. Nach der Zugabe der flüssigen Bestandteile ist ein deutlicher Kurvenanstieg zu erkennen. Erst wenn die Kurve wieder deutlich abfällt, ist die Weichmacheraufnahme abgeschlossen. Die Zeitdifferenz dieser beiden Punkte ist die Weichmacheraufnahmezeit (sog. Dryblendzeit). Das maximale Drehmoment wird vom Programm automatisch ausgewertet. Die Weichmacheraufnahme sowie das bei der Herstellung der Trockenmischungen bestimmte maximale Drehmoment sind in Tabelle 2 dargestellt.

**Tabelle 2:**

| Für die Aufnahme der flüssigen Rezepturkomponenten durch das vortemperierte PVC benötigte Zeit (Weichmacheraufnahme) und das bei der Herstellung der Trockenmischungen bestimmte maximale Drehmoment. | | | |
|---|---|---|---|
| Trockenmischung | A* | B** | C** |
| Weichmacheraufnahme [min.] | 9,2 | 11,1 | Nicht bestimmbar, Material bleibt feucht |
| Maximales Drehmoment [Nm] | 1,7 | 1,5 | Nicht bestimmbar |

| | | | |
|---|---|---|---|
| ** = Vergleichsbeispiel * = Erfindungsgemäß | | | |

Durch die mit der niedrigeren Zeit für die Weichmacheraufnahme verbundene kürzere Mischzeit ist die Verarbeitungsgeschwindigkeit der erfindungsgemäßen Mischung deutlich höher als die der Vergleichsrezeptur aus dem Stand der Technik. Das Terephthalat scheint unter den Messbedingungen nicht ausreichend vom PVC adsorbiert zu werden. Der Einsatz des erfindungsgemäßen Weichmachers ermöglicht es, Dryblends zur Verfügung zu stellen, die gegenüber dem Stand der Technik eine höhere Verarbeitungsgeschwindigkeit erlauben.

### Beispiel 6: Herstellung von Walzfellen und Pressplatten aus Dryblends

Aus den in Beispiel 5 beschriebenen Dryblends werden Walzfelle hergestellt. Die Herstellung der Walzfelle erfolgte auf einem Kalander W150 AP der Fa. Collin. Der Kalander der Fa. Collin besitzt einen automatischen Probenumleger und wird über einen zusätzlichen Ölthermostat (Fa. Single Typ: STO 1-6-12-DM) temperiert. Die Steuerung erfolgt über Software der Fa. Collin. Folgende Parameter wurden am Kalander eingestellt:
Walzentemperatur [°C]: 170
Walzzeit [min]: 5min

Fünfstufiges Programm zur Herstellung des Walzfelles
1. Stufe: Plastifizierung des Dryblends
2. Stufe: Änderung Walzenspalt
3. Stufe: Durchmischung Schmelze
4. Stufe: Walzfelloptimierung
5. Stufe: Walzfellabnahme

Nach dem Erreichen der Walzentemperatur wurde der Walzenspalt kalibriert. Zum Start der Messung wurde der Walzenspalt auf 0,2 mm eingestellt. Jeweils 160 Gramm eines Dryblends aus Beispiel 5 wurden eingewogen und bei stehenden Walzen in den Walzenspalt gegeben. Das Programm wurde gestartet. Die Walzen starteten mit einer Umdrehungszahl von 5 U/min und einer Friktion von 20 %. Nach ca. 1 min war die Plastifizierung zum größten Teil abgeschlossen und der Walzenspalt wurde auf 0,5 mm vergrößert. Es erfolgte eine 6malige Homogenisierung mittels automatischer Umlegeeinheit am Kalander. Nach 5 min wurde das Walzfell von der Walze entfernt und abgekühlt.

Aus dem Terephthalat haltigen Dryblend (Ester gemäß Beispiel 3, Dryblend C aus Beispiel 5) konnte weder unter den angegebenen Bedingungen noch bei einer um 10 °C erhöhten Temperaturen ein Walzfell hergestellt werden, da auf der Walze keine Plastifizierung erreicht werden konnte.

Daher wurde das Terephthalat haltige Dryblend (Beispiel C) in den weiteren Untersuchungen nicht mehr berücksichtigt.

### Herstellung der Pressplatten

Die Pressplatten wurden an einer Laborpresse der Fa. Collin hergestellt. Die vorgefertigten Walzfelle (siehe oben) wurden zur Herstellung der Pressplatten verwendet. Die Seitenränder der Walzfelle wurden mit Hilfe einer Schneidemaschine entfernt, das Walzfell wurde anschließend in ca. 14,5 x 14,5 cm große Stücke geschnitten. Für 1 mm dicke Pressplatten wurden je 2 Walzfellstücke in den 15 x 15 cm großen Pressrahmen aus Edelstahl gelegt.

Folgende Parameter wurden an der Laborpresse eingestellt:
Dreiphasiges Programm:
   Phase 1: Beide Platten 175°; Pressplattendruck: 5 bar; Phasenzeit: 60 Sekunden.
   Phase 2: Beide Platten 175°; Pressplattendruck: 200 bar; Phasenzeit: 120 Sekunden.
   Phase 3: Beide Platten 40°; Pressplattendruck: 200 bar; Phasenzeit: 270 Sekunden.

Die überschüssige Presslippe wurde nach Herstellung der Pressplatten entfernt.

### Beispiel 7:

### Bestimmung der weichmachenden Wirkung bzw. der Weichmachereffizienz an Pressplatten durch Bestimmung der Shore-Härte (Shore D)

Die Shore-Härte ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore Härte. Da in der Praxis Formulierungen / Rezepturen häufig auf eine bestimmte Shore Härte hin eingestellt bzw. optimiert werden, kann bei sehr effizienten Weichmachern demnach ein bestimmter Anteil in der Rezeptur eingespart werden, was eine Kostenreduktion für den Verarbeiter bedeutet.

Die Härte-Messungen wurden bei 25 °C nach DIN 53 505 mit einem Shore-D-Messgerät der Fa. Zwick-Roell durchgeführt (Aufeinanderlegen von 6 planparallelen 1 mm dicken Platten), der Messwert jeweils nach 3 Sekunden abgelesen. An jedem Probekörper (hergestellt gemäß Beispiel 6) wurden Messungen an drei verschiedenen Stellen durchgeführt, und ein Mittelwert gebildet.

Hierbei ist anzumerken, dass die Messung der Shore-Härte erst 24 Stunden nach Herstellung der Prüfkörper (Lagerung bei 25 °C) erfolgte. Die Ergebnisse der Härtebestimmung sind in Tabelle 3 zusammengestellt.

**Tabelle 3: Härte nach Shore D an Pressplatten hergestellt gemäß Beispiel 6**

| Prüfkörper aus Trockenmischung gemäß Bsp. | A* | B** | C** |
|---|---|---|---|
| Shore D | 42 | 44 | n.b. |

| | | | |
|---|---|---|---|
| ** = Vergleichsbeispiel * = Erfindungsgemäß | | | |

Es werden somit weichgemachte Kunststoffe zur Verfügung gestellt, die gegenüber dem als Vergleichsprodukt eingesetzten Phthalatweichmachern eine verbesserte Effizienz aufweisen und daher insbesondere zu niedrigeren Rezepturkosten führen.

### Beispiel 8: Flüchtigkeit des Weichmachers aus Prüfkörpern

Die Prüfkörper aus Beispiel 6 wurden bei 120 °C im Umluftwärmeschrank (Fa. Memmert) für 7 Tage gelagert. Vor jeder Wägung wurden die Proben eine Stunde im Exsikkator bei Raumtemperatur temperiert

Ergebnis (Mittelwert aus jeweils 6 Proben):

| | |
|---|---|
| Masseverlust Beispiel 1 (Furandicarboxylat): | 0,72 % |
| Masseverlust Beispiel 2 (Phthalat): | 0,85 % |

Die erfindungsgemäßen Produkte weisen gegenüber dem entsprechenden Vergleichsbeispiel C13-Phthalat eine geringere Flüchtigkeit auf. Somit würden entsprechende Weich-PVC-Produkte eine längere Lebensdauer aufweisen.

## Patentansprüche

1. C₁₁- bis C₁₃-Dialkylester der Furandicarbonsäure.

2. Weichmacher enthaltend mindestens zwei isomere C₁₁ bis C₁₃ Dialkylfuran-2,5-dicarboxylate.

3. Weichmacher nach Anspruch 2,
**dadurch gekennzeichnet,**
**dass** die isomeren C₁₁ bis C₁₃ Dialkylfuran-2,5-dicarboxylate isomere C₁₁ bis C₁₃ Alkylgruppen enthalten.

4. Weichmacher nach einem der Ansprüche 2 oder 3,
**dadurch gekennzeichnet,**
**dass** keiner der isomeren C₁₁ bis C₁₃ Dialkylfuran-2,5-dicarboxylate einen Anteil von mehr als 90 Gew.-% im Estergemisch besitzt.

5. Weichmacher nach einem der Ansprüche 2 bis 4,
**dadurch gekennzeichnet,**
**dass** die isomeren C₁₁ bis C₁₃ Alkylgruppen ausgewählt sind aus der Gruppe unverzweigte Alkylgruppe, einfach verzweigte Alkylgruppe, zweifach verzweigte Alkylgruppe, dreifach verzweigte Alkylgruppe, vierfach verzweigte Alkylgruppe oder Mischungen davon.

6. Weichmacher nach einem der Ansprüche 2 bis 5,
**dadurch gekennzeichnet,**
**dass** der Weichmacher Ditridecylfuran-2,5-dicarboxylat enthält.

7. Weichmacher nach einem der Ansprüche 2 bis 6,
**dadurch gekennzeichnet,**
**dass** er einen zusätzlichen Weichmacher ausgewählt aus der Gruppe Alkylbenzoate, Dialkyladipate, Glycerinester, Citronensäuretrialkylester, acylierte Citronensäuretrialkylester, Trialkylmellitate, Glykoldibenzoate, Dialkylterephthalate, Dialkylphthalate, Isosorbidester, Dialkylester der 1,2-, 1,3- oder 1,4-Cyclohexandicarbonsäure enthält.

8. Verfahren zur Herstellung eines Weichmachers nach einem der Ansprüche 2 bis 7, umfassend die Verfahrensschritte:
a) Umsetzung eines stöchiometrischen Überschusses von C₁₁-C₁₃-Alkoholen, optional in Gegenwart eines Katalysators, mit Furandicarbonsäure oder einem entsprechend geeigneten Derivat der Furandicarbonsäure
b) Abtrennen des überschüssigen Alkohols nach vollständiger Umsetzung der Furandicarbonsäure oder der des entsprechend geeigneten Derivats zum C₁₁-C₁₃-Dialkylester der Furandicarbonsäure und
c) Aufarbeitung des Reaktionsgemisches zur Gewinnung des erfindungsgemäßen Produktes in hoher Reinheit.

9. Verwendung der Weichmacher nach einem der Ansprüchen 2 bis 7 in Klebstoffen, Dichtungsmassen, Beschichtungsmassen, Lacken, Farben, Plastisolen, Pasten, Kunstleder, Fußbodenbeläge, Unterbodenschutz, Gewebebeschichtungen, Tapeten, Kabel und Drahtisolierungen, Folien, Automobilinnenanwendungen oder Tinten.

10. Verwendung der Weichmacher nach einem der Ansprüche 2 bis 7 als Weichmacher für Polymere , **dadurch gekennzeichnet,**
**dass** das Polymer ausgewählt ist aus: Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten und insbesondere PVC ist.

11. Verwendung der Weichmacher nach einem der Ansprüche 2 bis 7 als Weichmacher für Polymere, **dadurch gekennzeichnet**
**dass** das Polymer ein Copolymer von Vinylchlorid mit einem oder mehreren Monomeren ausgewählt aus der Gruppe bestehend aus Vinylidenchlorid, Vinylacetat, Vinylpropionat, Vinylbutyrat, Vinylbenzoat, Methylacrylat, Ethylacrylat, oder Butylacrylat ist.

## Claims

1. C₁₁ to C₁₃ dialkyl esters of furandicarboxylic acid.

2. Plasticizer comprising at least two isomeric C₁₁ to C₁₃ dialkyl furan-2,5-dicarboxylates.

3. Plasticizer according to Claim 2,
**characterized in that**
the isomeric C₁₁ to C₁₃ dialkyl furan-2,5-dicarboxylates contain isomeric C₁₁ to C₁₃ alkyl groups.

4. Plasticizer according to either of Claims 2 and 3,
**characterized in that**
none of the isomeric C₁₁ to C₁₃ dialkyl furan-2,5-dicarboxylates has a proportion of more than 90% by weight in the ester mixture.

5. Plasticizer according to any of Claims 2 to 4,
**characterized in that**
the isomeric C₁₁ to C₁₃ alkyl groups are selected from the group of unbranched alkyl group, singly branched alkyl group, doubly branched alkyl group, triply branched alkyl group, quadruply branched alkyl group and mixtures thereof.

6. Plasticizer according to any of Claims 2 to 5,
**characterized in that**
the plasticizer comprises ditridecyl furan-2,5-dicarboxylate.

7. Plasticizer according to any of Claims 2 to 6,
**characterized in that**
it comprises an additional plasticizer selected from the group of alkyl benzoates, dialkyl adipates, glyceryl esters, trialkyl citrates, acylated trialkyl citrates, trialkyl mellitates, glycol dibenzoates, dialkyl terephthalates, dialkyl phthalates, isosorbide esters, dialkyl esters of 1,2-, 1,3- or 1,4-cyclohexanedicarboxylic acid.

8. Process for preparing a plasticizer according to any of Claims 2 to 7,
comprising the process steps of:
a) reacting a stoichiometric excess of C₁₁-C₁₃-alcohols, optionally in the presence of a catalyst, with furandicarboxylic acid or a correspondingly suitable derivative of furandicarboxylic acid
b) removing the excess alcohol after complete conversion of the furandicarboxylic acid or of the correspondingly suitable derivative to the C₁₁-C₁₃-dialkyl ester of furandicarboxylic acid and
c) working up the reaction mixture to obtain the inventive product in high purity.

9. Use of the plasticizers according to any of Claims 2 to 7 in adhesives, sealing compounds, coating compositions, lacquers, paints, plastisols, pastes, synthetic leather, floor coverings, underbody protection, fabric coatings, wallpaper, cables and wire insulations, films, automobile interior applications or inks.

10. Use of the plasticizers according to any of Claims 2 to 7 as plasticizers for polymers,
**characterized in that**
the polymer is selected from: polyvinyl chloride (PVC), polyvinylidene chloride (PVDC), polyacrylates, especially polymethyl methacrylate (PMMA), polyalkyl methacrylate (PAMA), fluoropolymers, especially polyvinylidene fluoride (PVDF), polytetrafluoroethylene (PTFE), polyvinyl acetate (PVAc), polyvinyl alcohol (PVA), polyvinyl acetals, especially polyvinyl butyral (PVB), polystyrene polymers, especially polystyrene (PS), expandable polystyrene (EPS), acrylonitrile-styrene-acrylate (ASA), styrene acrylonitrile (SAN), acrylonitrile-butadiene-styrene (ABS), styrene-maleic anhydride copolymer (SMA), styrene-methacrylic acid copolymer, polyolefins, especially polyethylene (PE) or polypropylene (PP), thermoplastic polyolefins (TPO), polyethylene-vinyl acetate (EVA), polycarbonates, polyethylene terephthalate (PET), polybutylene terephthalate (PBT), polyoxymethylene (POM), polyamide (PA), polyethylene glycol (PEG), polyurethane (PU), thermoplastic polyurethane (TPU), polysulphides (PSu), biopolymers, especially polylactic acid (PLA), polyhydroxybutyral (PHB), polyhydroxyvaleric acid (PHV), polyesters, starch, cellulose and cellulose derivatives, especially nitrocellulose (NC), ethylcellulose (EC), cellulose acetate (CA), cellulose acetate/butyrate (CAB), rubber or silicones, and mixtures or copolymers of the polymers mentioned or monomeric units thereof, and more particularly is PVC.

11. Use of the plasticizers according to any of Claims 2 to 7 as plasticizers for polymers,
**characterized in that**
the polymer is a copolymer of vinyl chloride with one or more monomers selected from the group consisting of vinylidene chloride, vinyl acetate, vinyl propionate, vinyl butyrate, vinyl benzoate, methyl acrylate, ethyl acrylate and butyl acrylate.

## Revendications

1. Ester C₁₁-C₁₃-dialkylique de l'acide furannedicarboxylique.

2. Plastifiant contenant au moins deux furanne-2,5-dicarboxylates de C₁₁-C₁₃-dialkyle isomères.

3. Plastifiant selon la revendication 2, **caractérisé en ce que** les furanne-2,5-dicarboxylates de C₁₁-C₁₃-dialkyle isomères contiennent des groupes C₁₁-C₁₃-alkyle isomères.

4. Plastifiant selon l'une quelconque des revendications 2 ou 3, **caractérisé en ce qu'**aucun des furanne-2,5-dicarboxylates de C₁₁-C₁₃-dialkyle isomères ne représente une proportion supérieure à 90% en poids dans le mélange d'esters.

5. Plastifiant selon l'une quelconque des revendications 2 à 4, **caractérisé en ce que** les groupes C₁₁-C₁₃-alkyle isomères sont choisis dans le groupe formé par les groupes alkyle non ramifiés, les groupes alkyle monoramifiés, les groupes alkyle diramifiés, les groupes alkyle triramifiés, les groupes alkyle tétraramifiés ou leurs mélanges.

6. Plastifiant selon l'une quelconque des revendications 2 à 5, **caractérisé en ce que** le plastifiant contient du furanne-2,5-dicarboxylate de ditridécyle.

7. Plastifiant selon l'une quelconque des revendications 2 à 6, **caractérisé en ce qu'**il contient un plastifiant supplémentaire, choisi dans le groupe formé par les benzoates d'alkyle, les adipates de dialkyle, les esters de glycérol, les esters trialkyliques de l'acide citrique, les esters trialkyliques acylés de l'acide citrique, les trimellitates de trialkyle, les dibenzoates de glycol, les téréphtalates de dialkyle, les phtalates de dialkyle, les esters d'isosorbide, les esters dialkyliques de l'acide 1,2-cyclohexanedicarboxylique, de l'acide 1,3-cyclohexanedicarboxylique ou de l'acide 1,4-cyclohexanedicarboxylique.

8. Procédé pour la préparation d'un plastifiant selon l'une quelconque des revendications 2 à 7, comprenant les étapes de procédé consistant à :
a) transformer un excès stoechiométrique de C₁₁-C₁₃-alcools, éventuellement en présence d'un catalyseur, avec de l'acide furannedicarboxylique ou un dérivé correspondant approprié de l'acide furannedicarboxylique
b) séparer l'alcool en excès après transformation complète de l'acide furannedicarboxylique ou du dérivé correspondant approprié en ester C₁₁-C₁₃-dialkylique de l'acide furannedicarboxylique et
c) traiter le mélange réactionnel pour obtenir le produit selon l'invention à une pureté élevée.

9. Utilisation du plastifiant selon l'une quelconque des revendications 2 à 7 dans des adhésifs, des masses de bouchage, des masses de revêtement, des laques, des peintures, des plastisols, des pâtes, des cuirs synthétiques, des revêtements de sol, la protection des bas de caisse, des revêtements de tissus, des tapis, des câbles et des isolations de fils, des feuilles, des applications pour l'intérieur de véhicules ou des encres.

10. Utilisation du plastifiant selon l'une quelconque des revendications 2 à 7 comme plastifiant pour des polymères, **caractérisée en ce que** le polymère est choisi parmi le poly(chlorure de vinyle) (PVC), le poly(chlorure de vinylidène) (PVDC), les polyacrylates, en particulier le poly(méthacrylate de méthyle)(PMMA), le poly(méthacrylate d'alkyle) (PAMA), les polymères fluorés, en particulier le poly(fluorure de vinylidène) (PVDF), le polytétrafluoroéthylène (PTFE), le poly(acétate de vinyle) (PVAc), le poly(alcool vinylique) (PVA), les polyvinylacétals, en particulier le polyvinylbutyral (PVB), les polymères de polystyrène, en particulier le polystyrène (PS), le polystyrène expansible (EPS), l'acrylonitrile-styrène-acrylate (ASA), le styrène-acrylonitrile (SAN), l'acrylonitrile-butadiène-styrène (ABS), le copolymère de styrène-anhydride de l'acide maléique (SMA), le copolymère de styrène-acide méthacrylique, les polyoléfines, en particulier le polyéthylène (PE) ou le polypropylène (PP), les polyoléfines thermoplastiques (TPO), le polyéthylène-acétate de vinyle (EVA), les polycarbonates, le poly(téréphtalate d'éthylène) (PET), le poly(téréphtalate de butylène) (PBT), le polyoxyméthylène (POM), le polyamide (PA), le polyéthylèneglycol (PEG), le polyuréthane (PU), le polyuréthane thermoplastique (TPU), les polysulfures (PSu), les biopolymères, en particulier le poly(acide lactique) (PLA), le polyhydroxybutyral (PHB), le poly(acide hydroxyvalérianique) (PHV), le polyester, l'amidon, la cellulose et les dérivés de cellulose, en particulier la nitrocellulose (NC), l'éthylcellulose (EC), l'acétate de cellulose (CA), l'acétate/butyrate de cellulose (CAB), les gommes ou les silicones ainsi que les mélanges ou les copolymères des polymères mentionnés ou de leurs motifs monomères et en particulier le PVC.

11. Utilisation du plastifiant selon l'une quelconque des revendications 2 à 7 comme plastifiant pour des polymères, **caractérisée en ce que** le polymère est un copolymère de chlorure de vinyle avec un ou plusieurs monomères choisis dans le groupe constitué par le chlorure de vinylidène, l'acétate de vinyle, le propionate de vinyle, le butyrate de vinyle, le benzoate de vinyle, l'acrylate de méthyle, l'acrylate d'éthyle ou l'acrylate de butyle.
